# EUROPEAN PATENT SPECIFICATION

(11) **EP 2 641 598 B1**
(45) Date of publication and mention of the grant of the patent: **06.06.2018**
(21) Application number: 11841251.9
(22) Date of filing: 15.11.2011
(51) Int. Cl.: A61K 31/24, A61K 31/80, A61K 38/43, A61P 1/00, A61K 9/16, A61K 9/20

(54) **ORALLY ADMINISTERED PHARMACEUTICAL COMPOSITION FOR THE TREATMENT OF IRRITABLE BOWEL SYNDROME, COMPRISING AN INTESTINAL MOTILITY MODIFIER, AN AGENT THAT PREVENTS GAS RETENTION, AND DIGESTIVE ENZYMES, AND PREPARATION METHOD THEREOF**
ORAL VERABREICHBARE PHARMAZEUTISCHE ZUSAMMENSETZUNG ZUR BEHANDLUNG DES REIZDARMSYNDROMS MIT EINEM MODIFIKATOR DER INTESTINALEN BEWEGLICHKEIT, EINEM MIITTEL ZUR VERHINDERUNG VON GASRÜCKHALTUNG SOWIE VERDAUUNGSENZYMEN UND VERFAHREN ZU IHRER HERSTELLUNG
COMPOSITION PHARMACEUTIQUE DESTINÉE À L'ADMINISTRATION PAR VOIE ORALE ET UTILE POUR LE TRAITEMENT DU SYNDROME DE L'INTESTIN IRRITABLE, CONSTITUÉE D'UN MODIFICATEUR DE LA MOTILITÉ INTESTINALE, D'UN AGENT EMPÊCHANT LA RÉTENTION DES GAZ ET D'ENZYMES DIGESTIVES, PROCÉDÉ DE PRÉPARATION DE LADITE COMPOSITION

(30) Priority: 16.11.2010 MX 2010012479
(43) Date of publication of application: 25.09.2013
(73) Proprietor: Posi Visionary Solutions LLP, London WC1R 4HE (GB)
(72) Inventor: BERNARDO ESCUDERO, Roberto, Hidalgo (MX); SAVOIR VILBOUEF, John Claude, México, D.F (MX)
(74) Representative: Abel & Imray
(86) International application number: PCT/MX2011/000138
(87) International publication number: WO 2012/067481

(56) References cited:
- WO-A1-95/01803
- WO-A1-95/01803
- KR-A- 20040 003 112
- US-A1- 2003 007 962
- US-A1- 2003 007 962
- US-A1- 2004 092 511
- US-A1- 2007 020 249
- US-A1- 2008 038 336
- US-A1- 2008 038 336
- DATABASE WPI Derwent Publications Ltd., London, GB; AN 2004-363191, XP055105462 & KR 2004 0 003 112 A (SEOUL PHARM CO LTD) 13 January 2004

## Description

### SCOPE OF THE INVENTION

This invention involves a pharmaceutical composition and its preparation in the form of a tablet, coated tablet, or capsule to be used in the treatment of irritable bowel syndrome, also known as irritable colon syndrome, based on: an intestinal motility modifier, an agent that prevents gas retention, a digestive enzyme, a binding agent, a diluting agent, an adsorbent, a disintegrant, a lubricant, and a glidant.

### BACKGROUND

Enzymes as medications have two important features that distinguish them from other types of drugs. First, enzymes normally bind and act upon their substrates with high affinity and specificity; second, enzymes are catalytic molecules, meaning they decrease the activation energy of a determined reaction, through which they convert multiple white molecules (substrates) into the desired products. The two aforementioned features make pharmaceutical enzymes potent and specific so they can carry out a therapeutic biochemical activity in the body that small molecules cannot; as a result, scientists have worked on the development of various enzymes for use as therapeutic agents. This concept of therapeutic enzymology already existed as substitution therapy for use in cases of genetic deficiencies in the 1960s. In 1987 the Food and DrugAdministration approved the first drug containing a recombinant enzyme, Activase® (alteplase, a recombinant human tissue plasminogen activator) for treatment of heart attacks caused by a clot blocking a coronary artery. In 1990, Adagen®, a form of bovine adenosine deaminase (BAD) treated with polyethylene glycol was approved for use in patients with a type of severe combined immunodeficiency (SCID), which is caused by chronic BAD deficiency. In 1994 Ceredase® was approved, the first enzyme replacement therapy with a recombinant enzyme, for the treatment of Gaucher's disease, related to lysosomal storage disease caused by glucocerebrosidase deficiency. Sacarosidase, a fructohydrolase β-fructofuranoside obtained from *Saccharomyces cerevisiae* yeast, is used in the treatment of congenital sucrase-isomaltase enzyme deficiency (CSID) in which patients are incapable of metabolizing sucrose. In the case of phenylketonuria, a genetic disease caused by reduced or non-existent activity of the phenylalanine hydroxylase enzyme, which converts phenylalanine into tyrosine, an oral treatment is being used in oral treatment based on the phenylalanine ammonialyase enzyme that is derived from a yeast, which degrades phenylalanine in the gastrointestinal tract. Another enzyme, a peptidase, is used in an oral formulation as a supplemental therapy in cases of Celiac's disease, a disorder of the small intestine caused by an autoimmune system reaction to the protein gliadin, which is found in products derived from wheat (Vellard, Michael. The enzyme as a drug: application of enzymes as pharmaceuticals. Current Opinion in Biotechnology.2003. Vol. 14: 444 - 450). The hydrolytic enzyme α-D-galactosidase, used in the treatment of gastrointestinal disorders, transforms non-absorbable oligosaccharides in the intestinal tract to prevent them from being fermented by intestinal bacterial flora (a gas-producing process); in reducing intestinal gas production, visceral distention is decreased and therefore, symptoms like distention, abdominal pain and flatulence decrease as well (http://www.beanogas.com accessed on April 28, 2009). α-D-galactosidase hydrolyzes three complex carbohydrates: raffinose, stachyose and verbascose to transform them into monosaccharides: Glucose, galactose and fructose and into the disaccharide: sucrose (whose hydrolysis is instantaneous during normal digestion). The α-D-galactosidase enzyme is not normally produced by human beings, for which reason raffinose, stachyose and verbascose arrive intact at the colon, where they are fermented by bacterial flora in a chemical reaction that produces hydrogen and methane (gas). Administration of the enzyme with food breaks up these three oligosaccharides before they arrive at the colon, preventing fermentation and gas production. The α-D-galactosidase that is used as a medication comes from the non-toxic food-grade fungus *Aspergillusniger*(http://www.beanogas.com accessed on April 28, 2009). Various other enzymes exist that are used medicinally for digestive disorders, among them amylase, β-D-galactosidase, cellulase, hemicellulase, lipase, papain, pepsin, rutin, chymotrypsin and trypsin.

Irritable bowel syndrome (IBS), previously known as irritable colon syndrome, is a functional disorder of the intestine, characterized by symptoms of abdominal discomfort or pain that are associated with changes in bowel habits. IBS is currently understood to be a result of interactions between many factors that contribute to the onset of symptoms, rather than as a singular disease. There is no single physiopathological mechanism that can explain it but there are at least 3 interrelated factors that act in ways that vary from person to person.

The factors are:
i) Changed intestinal reactivity, ii) motility or secretion in response to provocative luminal stimuli (food, distention, inflammation, bacterial factors) or environmental stimuli (psycho-social stress) that result in symptoms of diarrhea or constipation and iii) bowel hypersensitivity with increased visceral perception and pain.

Changes in regulation of the "brain-intestine" axis.

Diagnosis of IBS is based on identifying positive symptoms, called the Rome III Diagnostic Criteria (Longstreth, G.F. 2006. Functional bowel disorders. Gastroenterology. Vol. 130, No. 5:1480-91), and on ruling out other intestinal tract diseases with similar manifestations. These criteria are:
Recurring abdominal discomfort or pain for at least three days per month for the last three months, associated with two or more of the following conditions: a) improvement with defecation, b) onset associated with a change in the frequency of bowel movements and c) onset associated with a change in the appearance of stool.

In which discomfort refers to a disagreeable sensation not described as pain.

The criteria must have been fulfilled in the last three months, with onset of symptoms at least six months before diagnosis.

IBS is one of the most common medical disorders in the world, occurring more frequently in women aged 30 to 50, with prevalence in Latin America between 9 and 18% (Schmulson, Max J. 2008. Limited diagnostic screening can decrease the direct economic impact of irritable bowel syndrome (IBS). Rev Med Chile. Vol. 136: 1398 - 1405).
The symptom pattern in Mexico is IBS with constipation; abdominal distention is a common symptom in this pattern of the disease. In the Mexican population, abdominal distention and gas are reported as symptoms with high frequency. Irritable bowel syndrome is a real pathological condition that has significant impact on those who suffer from it (symptom severity, functional impairment, diminished quality of life), in addition to constituting a significant economic burden for society and the state, in terms of the costs of medical care and absences from work (American Gastroenterological Association; 2002; American Gastroenterological Association position statement; "irritable bowel syndrome. Gastroenterology". Vol. 123, No. 6:2105-7).

There is no ideal or standard treatment for this disease. Trimebutine maleate, commonly known as trimebutine, has been used since 1969 as treatment for functional bowel disorders, including irritable bowel syndrome. Its principal effects are regularization of intestinal motility and an elevated threshold for pain caused by visceral distention (Roman F. J., et al. 1999. Pharmacological properties of trimebutine and N-monodesmethyltrimebutine. J Pharmacol Exp Ther. Vol. 289, No. 3:1391- 1397).

Abdominal pain, distention and flatulence represent very common symptoms in functional bowel disorders, including irritable bowel syndrome, but their physiopathology and treatment have not been completely explained. Patients frequently associate these symptoms with excessive gas production in the bowel and reduction of the latter could represent an effective strategy for symptomatic improvement in irritable bowel syndrome, for which simethicone has been used. Simethicone is an inert silicon that acts directly on the surface tension of gastrointestinal mucous, thus affecting the formation of bubbles in the digestive tract, destroying them and encouraging the confluence of small bubbles into bigger bubbles, which translates into the prevention of gas retention and the associated discomforts. It is important to note that these symptoms may be produced or worsened in a patient with irritable bowel syndrome, not only by an increase in gas production, but also by the "normal" presence of gas in the digestive tube coupled with increased visceral sensitivity. Strategies do currently exist for the treatment of this problem, such as the use ofactivated carbon,dietary restriction and probiotic consumption; however, none of these are ideal and the results obtained are contradictory in each case. In this context, the breakdown of non-absorbable oligosaccharides, found in legumes, fruits and vegetables, before they reach the colon (where they will be fermented by bacterial flora and will produce gas) may represent an attractive alternative. Administration of α-D-galactosidase may achieve this effect (Di Stefano M., et al. 2007; "The effect of oral alpha-galactosidase on intestinal gas production and gas-related symptoms". Dig Dis Sci. January. Vol. 52, No. 1:78-83).

There are pharmaceutical products that modify intestinal motility for use with intestinal disorders, such as:
Trimebutine and its salts.
Fenoverine.
Mebeverine.
Dicycloverine.
Pinaverium bromide.
Alosetron.
Tegaserod.
Loperamide.
Floroglucinol.
Trimetilfloroglucinol.
Butylscopolamine.
Pargeverine.

All of these can be used in combination with simethicone to obtain a pharmaceutical formulation for oral administration to be used in intestinal disorders, as is the specific case with irritable bowel syndrome.

There are also various enzymes with physiological activity that are useful in the treatment of intestinal disorders, such as: α-D-galactosidase, amylase, cellulase, hemicellulase, lipase, papain, rutin, chymotrypsin and trypsin.

All of the aforementioned enzymes can be used in combination with simethicone and with intestinal motility modifiers to obtain a pharmaceutical formulation for oral administration to be used in intestinal disorders, as is the specific case with irritable bowel syndrome.

The combination of trimebutine and its salts, a regulating agent of intestinal motility with analgesic properties, simethicone, an agent that prevents gas retention, and an enzyme or enzyme combination, results in an effective treatment for symptomatology reduction in patients with irritable bowel syndrome.

Considering that trimebutine acts upon Auerbach's(muscular) and Meissner's (submucosal) plexus specifically, it acts upon the enkephalinergic receptors responsible for regulating peristaltic movements. Trimebutine acts as much on hypermotility as on hypomotility, depressing or elevating peristalsis and leading to normalization of intestinal transit. Trimebutine also has analgesic(modulation of visceral sensitivity), antispasmodic and antiemetic properties (Delvaux M. & Wingate D. 1997. Trimebutine: "Mechanism of action, effects on gastrointestinal function and clinical results". J Int Med Res. Vol. 25, No. 5:225-46).

Among the solutions that have been proposed to treat IBS symptomatology, the paper WO2001/047515 reports the use of trimebutine alone, to develop a useful medication to treat somatic pain and abdominal inflammation; however, it only focuses on symptom relief for this ailment.

Similarly, numerous papers exist concerning the treatment of inflammation, abdominal pain and ailments associated with IBS; however, treatment of IBS at its source has not been resolved in any of said papers as shown by the following citations:
The paper MXPA02006376 refers to the use of trimebutine alone to prevent or treat somatic pain and inflammation associated with gastric ailments; however, when looking to alleviate symptoms, pain is not eradicated as a function of its causal agent.

The paper US 2003/0119903 reports the use of trimebutine alone to prepare a medication to treat somatic inflammatory pain as well as chronic pain associated with gastric ailments.

The paper US 2004/0009234 reports a pharmaceutical composition and associated treatment to prevent gastrointestinal disorders, making use of trimebutine alone without achieving the desired end result of combatting the origin of these ailments.

The paper MX00PA05010821A reports the use of trimebutine to treat constipation, without achieving the desired end result of combating the origin of these ailments.

The paper WO1995001803 reports the use of trimebutine to treat gastrointestinal pain and disorders such as indigestion caused by excessive food intake, gastro-esophageal reflux, dyspepsia and constipation without achieving the desired end result of combating the source of these ailments.

The paper WO95001784 reports the use of a pharmaceutical composition for treating and alleviating indigestion, heartburn and other gastrointestinal disorders using famotidine, sucralfate, simethicone and α-D-galactosidase; however, the composition of the specified publication lacks an agent that effectively promotes rapid gastric emptying, which makes it inefficient in the treatment of IBS, as the patient who is unable to defecate quickly will not have a sensation of relief.

The paper US 2008/0038240 reports the use of enzymes to improve absorption of carbohydrates in humans, avoiding the formation of intestinal gases.

The paper US 4447412 reports an enzymatic composition for the treatment of digestive dysfunction, composed of pancreatic and proteolytic enzymes.

The paper US 4079125 reports an extended-release enzymatic composition able to withstand several hours of exposure to gastric fluids, protecting the biological activity of the enzymes and releasing them after 5 - 30 minutes of exposure to intestinal fluids.

The papers US 5460812 and US 324514 report the use of enzymes in the treatment of digestive disorders.

One objective of this invention is to provide a pharmaceutical composition for oral administration with application in intestinal disorders based on an intestinal motility modifier, an agent that prevents gas retention, digestive enzymes, a binding agent, a diluting agent, an adsorbent, a disintegrant, a lubricant, and a glidant.

Another objective of this invention is to provide a pharmaceutical formulation for oral administration with application in intestinal disorders based on an intestinal motility modifier, an agent that prevents gas retention, digestive enzymes, a binding agent, a diluting agent, an adsorbent, a disintegrant, a lubricant, and a glidant that is effective in normalizing intestinal transit.
Another objective of this invention is to provide a pharmaceutical formulation for oral administration with application in intestinal disorders based on an intestinal motility modifier, an agent that prevents gas retention, digestive enzymes, a binding agent, a diluting agent, an adsorbent, a disintegrant, a lubricant, and a glidant that is effective in achieving analgesic activity in the treatment of gastrointestinal ailments.

Another objective of this invention is to provide a pharmaceutical formulation for oral administration with application in intestinal disorders based on an intestinal motility modifier, an agent that prevents gas retention, digestive enzymes, a binding agent, a diluting agent, an adsorbent, a disintegrant, a lubricant, and a glidant that is effective in achieving antispasmodic activity.

A final objective of this invention is to provide a pharmaceutical composition or formulation for oral administration with application in intestinal disorders based on an intestinal motility modifier, an agent that prevents gas retention, digestive enzymes, a binding agent, a diluting agent, an adsorbent, a disintegrant, a lubricant, and a glidant that is effective in reducing symptoms related to intestinal gas such as distention, pain and flatulence.

### DETAILED DESCRIPTION OF THE INVENTION

The pharmaceutical formulation is prepared in the form of a tablet, coated tablet, or capsule, for use in irritable bowel syndrome, also known as irritable colon syndrome, based on an intestinal motility modifier, an agent that prevents gas retention and digestive enzymes.

The intestinal motility modifier, the agent that prevents gas retention, the digestive enzyme, the binding agent, the diluting agent, the disintegrant, the lubricant, and the glidant, are mixed.

A binder solution is prepared.

The intestinal motility modifier, the enzyme α-D-galactosidase, the binding agent, the diluting agent, the disintegrant, the lubricant, and the glidant are sifted in order to break up any clumps.

All of the substances mentioned in the previous step are mixed and then moistened with the binder solution.

The product resulting from the previous step is ground, dried and sifted.

If the final composition is solid, the mixture is compressed to form a tablet or a coated tablet; otherwise capsules are prepared.

The tablets or capsules are packaged in packing material.

To carry out the specified manufacturing process, one will use the equipment that is conventionally used in the production of a pharmaceutical formulation with the indicated characteristics. All of the raw materials used are of pharmaceutical grade. Below, some practical examples of how the formulations were prepared are detailed for illustrative, but not restrictive, purposes.

### Examples

An example of tablet formulation of Trimebutine Maleate/α-D-galactosidase/Simethicone obtained by wet granulation.

| Component | Amount |
|---|---|
| Trimebutine maleate | 200.000 mg |
| Simethicone | 75.000 mg |
| α-D-galactosidase | 90.000 mg* |
| Pregelatinized starch | 75.000 mg |
| Lactose hydrous | 105.000 mg |
| Croscarmellose sodium | 30.000 mg |
| Microcrystalline cellulose | 115.000 mg |
| Dibasic calcium phosphate | 300.000 mg |
| Magnesium stearate | 10.000 mg |

| | |
|---|---|
| *90 mg is equivalent to 450 U/gal. U/Gal considering a raw material of α-D-galactosidase with enzymatic activity of 5,,000 U/gal per gram. | |

Procedure for manufacturing tablets of Trimebutine Maleate/α-D-galactosidase/Simethicone by wet granulation.
1. Prepare a binder solution by dispersing 20% of the pregelatinized starch in a sufficient amount of water.
2. Pass the following raw materials through a sieve with mesh size of 420 to 2,000 microns:
   - The rest of the pregelatinized starch (80%)
   - The α-D-galactosidase
   - Trimebutine maleate
   - Lactose hydrous
   - Croscarmellose sodium
   - Dibasic calcium phosphate
3. Add the dibasic calcium Phosphate and the pregelatinized starch (80%) into the mixer/granulator and mix for 5 to 20 minutes at 50 to 200 rpm.
4. At the end of this mixing and without stopping the stirring, manually add the simethicone in "string" form over a time period not to exceed 15 minutes.
5. Add the Trimebutine Maleate, α-D-galactosidase, Lactose hydrous and Croscarmellose sodium to the mixer and mix for 5 to 20 minutes at 50 to 200 rpm.
6. Moisten with the binder solution from step 1.
7. Pass the product obtained from the grinder in step 6 through a sieve with openings from 3,000 to 5,000 microns.
8. Dry the product at a temperature of 30 to 60°C until it reaches a residual humidity of 1.0 - 3.0%.
9. Grind the product obtained in step 8 through a grinder with a sieve from 0.033 to 0.094 inches and at a speed of 500 to 1,500 rpm.
10. Pass the microcrystalline Cellulose and the magnesium stearate through a sieve with a mesh size from 420 to 2,000 microns.
11. Add the following products to the mixer:
   The granules obtained in step 9;
   the microcrystalline Cellulose obtained in step 10 and mix for 10 to 30 minutes at 15 to 30 rpm.
12. Add the magnesium stearate obtained in step 9 to the mixer and mix for 5 to 10 minutes at 15 to 30 rpm.
13. Compress the product obtained in step 12.
An example of the formulation of Trimebutine Maleate/α-D-galactosidase/Simethicone tablets obtained by direct compression.

| Component | Amount |
|---|---|
| Trimebutine maleate | 200,000 mg |
| Simethicone | 75,000 mg |
| α-D-galactosidase | 90,000 mg* |
| Croscarmellose sodium | 30,000 mg |
| Microcrystalline cellulose | 210,000 mg |
| Magnesium aluminometasilicate | 310,000 mg |
| Magnesium stearate | 10,000 mg |

| | |
|---|---|
| *90 mg are equivalent to 450 U/gal. U/Gal considering a raw material of α-D-galactosidase with enzymatic activity of 5.000 U/gal per gram. | |

Example Procedure for the manufacture of Trimebutine Maleate/α-D-galactosidase/Simethicone tablets by direct compression.
1. Pass the following raw materials through a sieve with mesh size of 420 to 2,000 microns:
   - The α-D-galactosidase
   - Trimebutine maleate
   - Microcrystalline cellulose
   - Croscarmellose sodium
   - Dibasic calcium phosphate
2. Add the magnesium Aluminometasilicate to a mixer and begin stirring at a speed between 40 and 100 rpm. Without stopping the stirring, manually add the Simethicone in "string" form very gradually over a time not to exceed 30 minutes (Mixture A).
3. Add the following products to a mixer:
   Half of mixture A from step 2
   Half of the microcrystalline Cellulose
   Half of the Trimebutine maleate
   The α-D-galactosidase
   The Croscarmellose sodium
   The rest of the Trimebutine maleate
   The rest of the microcrystalline Cellulose
   The rest of mixture A
   And mix for 10 to 30 minutes at 15 to 30 rpm (mixture B)
4. Pass the magnesium stearate through a sieve with mesh size of 420 to 2,000 microns.
5. Add the magnesium stearate obtained in step 4 to mixture B and mix for 5 to 10 minutes at 15 to 30 rpm.
6. Compress the product obtained in step 5.

An example of manufacturing Trimebutine Maleate/α-D-galactosidase/Simethicone tablets obtained through dry granulation.

| Component | Amount |
|---|---|
| Trimebutine maleate | 200.000 mg |
| Simethicone | 75.000 mg |
| α-D-galactosidase | 90.000 mg* |
| Hydroxypropyl cellulose | 50.000 mg |
| Lactose hydrous | 110.000 mg |
| Crospovidone | 30.000 mg |
| Microcrystalline cellulose | 125.000 mg |
| Dibasic calcium phosphate | 310.000 mg |
| Magnesium stearate | 10.000 mg |

| | |
|---|---|
| *90 mg are equivalent to 450 U/gal. U/Gal considering a raw material of α-D-galactosidase with enzymatic activity of 5000 U/gal per gram. | |

Example Procedure for the manufacture of Trimebutine Maleate/α-D-galactosidase/Simethicone tablets by dry granulation.
1. Pass the following raw materials through a sieve with mesh size of 420 to 2,000 microns:
- The hydroxypropyl cellulose
- The α-D-galactosidase
- Trimebutine maleate
- Lactose hydrous
- 50% of the Crospovidone
   - Dibasic Calcium Phosphate.

2. Incorporate the Dibasic Calcium Phosphate and the hydroxpropyl cellulose to the granulating mixing equipment and mix for 5 and 20 minutes at 50 to 200 rpm.
3. After mixing and without stopping stirring, manually add the simethicone in "string" form for no longer than 30 minutes.
4. Add Trimbutine Maleate, α-D-galactosidase, Microcrystalline Cellulose, 50% of the Crospovidone to the mixer and mix between 5 and 20 minutes at 50 to 200 rpm.
5. Compress the product obtained in step 4.
6. Grind the product obtained in step 5 with the granulating equipment with a mesh size of 1,180 to 2,000 microns.
7. Compress the granules that were obtained in step 6 again.
8. Grind the product obtained in step 7 with the granulating equipment with a mesh size of 1,400 to 1,700 microns.
9. Pass the 50% of the Crospovidone, the microcrystalline cellulose, and the magnesium Stearate through a sieve with a mesh size of 420 to 2,000 microns,
10. Add the following products to the mixer:
The granules obtained in step 8.
50% of the Croscarmellose Sodium from step 9.
The Microcrystalline cellulose obtained in step 9
And mix for 10 to 30 minutes at 15 to 30 rpm.
11. Add the magnesium stearate obtained in step 9 to the mixer and mix for 5 to 10 minutes at 15 to 30 rpm.
12. Compress the product obtained in step 11. Below are the excipients which can adequately perform the indicated functions:

| Function | Excipient |
|---|---|
| Binding Agent | Hydroxypropyl cellulose, corn starch, propyl cellulose, methyl cellulose. |
| Diluting Agent | Lactose, Microcrystalline cellulose, mannitol, sucrose |
| Absorbing Agent | Dibasic calcium phosphate, aluminum and magnesium silicate, colloidal silicon dioxide, microcrystalline cellulose |
| Disintegrating Agent | Croscarmellose sodium, corn starch, crospovidone |
| Lubricating Agent | Magnesium stearate, talc, stearic acid |
| Gliding Agent | Colloidal Silicon Dioxide |

- The diluting agent is selected from the excipients that have the function of increasing the apparent volume of the powder, and as such, increase the weight of the pill or capsule.
- The absorbing agent is selected from the excipients that are able to absorb certain amounts of liquid in an apparently dry condition.
- The disintegrating agent is selected from the excipients that are able to break (disintegrate) the pill and the granules when they come into contact with a liquid.
- The lubricating agent is selected from the excipients that are able to reduce the friction between the granules and the wall of the matrix during the process of compression or filling of the capsules.
- The gliding agent is selected from the excipients that are able to provide a flow to the granules of the hopper to the cavity of the matrix through the reduction of inter-particle friction.
- The binding agent is selected from the excipients that provide cohesiveness to the materials in powder form, forming granules.

## Claims

1. A pharmaceutical composition or formulation adapted for oral administration in tablet, coated tablet or capsule form for use in the treatment of intestinal disorders, the formulation comprising: an intestinal motility modifier, an agent which prevents the retention of gases, a digestive enzyme, a binding agent, a diluting agent, an absorbing agent, a disintegrating agent, a lubricating agent and a gliding agent; wherein
- the intestinal motility modifier is trimebutine or a pharmaceutically acceptable salt thereof,
- the agent which prevents the retention of gases is simethicone, and
- the enzyme is α-D-galactosidase.

2. The pharmaceutical formulation for use in accordance with claim 1, wherein the binding agent is selected from the group consisting of hydroxypropyl cellulose, corn starch, propyl cellulose and methyl cellulose.

3. The pharmaceutical formulation for use in accordance with claim 1 or claim 2, wherein the diluting agent is selected from the group consisting of lactose, microcrystalline cellulose, dibasic calcium phosphate and mannitol.

4. The pharmaceutical formulation for use in accordance with any one of claims 1 to 3, wherein the absorbing agent is selected from the group consisting of dibasic calcium phosphate, aluminum and magnesium silicate, colloidal silicon dioxide and microcrystalline cellulose.

5. The pharmaceutical formulation for use in accordance with any one of claims 1 to 4, wherein the disintegrating agent is selected from the group consisting of croscarmellose sodium, corn starch and crospovidone.

6. The pharmaceutical formulation for use in accordance with any one of claims 1 to 5, wherein the lubricating agent is selected from the group consisting of magnesium stearate, talc and stearic acid.

7. The pharmaceutical formulation for use in accordance with any one of claims 1 to 6, wherein the gliding agent is colloidal silicon dioxide.

8. The pharmaceutical formulation for use in accordance with any one of claims 1 to 7, wherein the digestive enzyme is α-D-galactosidase with an enzymatic energy of 450 Ugal.

9. A process for preparing a pharmaceutical composition of any one of claims 1 to 8, comprising:
mixing the intestinal motility modifier, the agent which prevents the retention of gases and the digestive enzyme, with a binding solution to humidify the intestinal motility modifier and digestive enzyme; grinding, drying and sieving the mix; obtaining a pharmaceutical formulation adapted for oral administration.

10. The process in accordance with claim 9, wherein the components are dried at a temperature of 30 to 60°C and the composition reaches a final residual humidity no greater than 5%.

11. The process in accordance with claim 10, wherein the mix is sieved through a mesh size of 420 to 2,000 microns.

## Patentansprüche

1. Pharmazeutische Zusammensetzung oder Formulierung, die zur oralen Verabreichung in Form einer Tablette, Filmtablette oder Kapsel geeignet ist, zur Verwendung bei der Behandlung von Darmerkrankungen, wobei die Formulierung umfasst: einen Darmmotilitätsmodifikator, ein Mittel, das die Retention von Gasen verhindert, ein Verdauungsenzym, ein Bindemittel, ein Verdünnungsmittel, ein Absorptionsmittel, ein Sprengmittel, ein Schmiermittel und ein Gleitmittel; wobei
- der Darmmotilitätsmodifikator Trimebutin oder ein pharmazeutisch annehmbares Salz davon ist,
- das Mittel, das die Retention von Gasen verhindert, Simethicon ist, und
- das Enzym α-D-Galactosidase ist.

2. Pharmazeutische Formulierung zur Verwendung nach Anspruch 1, wobei das Bindemittel aus der Gruppe bestehend aus Hydroxypropylcellulose, Maisstärke, Propylcellulose und Methylcellulose ausgewählt ist.

3. Pharmazeutische Formulierung zur Verwendung nach Anspruch 1 oder Anspruch 2, wobei das Verdünnungsmittel aus der Gruppe bestehend aus Lactose, mikrokristalliner Cellulose, dibasischem Calciumphosphat und Mannit ausgewählt ist.

4. Pharmazeutische Formulierung zur Verwendung nach irgendeinem der Ansprüche 1 bis 3, wobei das Absorptionsmittel aus der Gruppe bestehend aus dibasischem Calciumphosphat, Aluminium- und Magnesiumsilicat, kolloidalem Siliciumdioxid und mikrokristalliner Cellulose ausgewählt ist.

5. Pharmazeutische Formulierung zur Verwendung nach irgendeinem der Ansprüche 1 bis 4, wobei das Sprengmittel aus der Gruppe bestehend aus Croscarmellose-Natrium, Maisstärke und Crospovidon ausgewählt ist.

6. Pharmazeutische Formulierung zur Verwendung nach irgendeinem der Ansprüche 1 bis 5, wobei das Schmiermittel aus der Gruppe bestehend aus Magnesiumstearat, Talkum und Stearinsäure ausgewählt ist.

7. Pharmazeutische Formulierung zur Verwendung nach irgendeinem der Ansprüche 1 bis 6, wobei das Gleitmittel kolloidales Siliciumdioxid ist.

8. Pharmazeutische Formulierung zur Verwendung nach irgendeinem der Ansprüche 1 bis 7, wobei das Verdauungsenzym α-D-Galactosidase mit einer enzymatischen Aktivität von 450 Ugal ist.

9. Verfahren zur Herstellung einer pharmazeutischen Zusammensetzung nach irgendeinem der Ansprüche 1 bis 8, umfassend:
Mischen des Darmmotilitätsmodifikators, des Mittels, das die Retention von Gasen verhindert, und des Verdauungsenzyms mit einer Bindelösung zur Befeuchtung des Darmmotilitätsmodifikators und des Verdauungsenzyms; Mahlen, Trocknen und Sieben der Mischung; Erhalten einer pharmazeutischen Formulierung, die zur oralen Verabreichung geeignet ist.

10. Verfahren nach Anspruch 9, bei dem die Komponenten bei einer Temperatur von 30 bis 60°C getrocknet werden und die Zusammensetzung eine Endrestfeuchte von nicht mehr als 5% erreicht.

11. Verfahren nach Anspruch 10, bei dem die Mischung durch eine Maschenweite von 420 bis 2.000 Mikrometern gesiebt wird.

## Revendications

1. Une composition ou formulation pharmaceutique adaptée pour une administration orale sous forme de comprimé, de comprimé enrobé ou de capsule destinée à être utilisée dans le traitement de troubles intestinaux, la formulation comprenant un modificateur de motilité intestinale, un agent qui empêche la rétention des gaz, une enzyme digestive, un agent de liaison, un agent de dilution, un agent absorbant, un agent de désintégration, un agent lubrifiant et un agent de glissement, dans laquelle:
- le modificateur de motilité intestinale est la trimébutine ou ses sels pharmaceutiques acceptables ;
- l'agent qui empêche la rétention des gaz est la siméthicone ;
- l'enzyme est l'α-D-galactosidase.

2. La formulation pharmaceutique pour son utilisation selon la revendication 1, dans laquelle l'agent de liaison est choisi dans le groupe comprenant l'hydroxypropylcellulose, l'amidon de maïs, la propylcellulose et la méthylcellulose.

3. La formulation pharmaceutique pour son utilisation selon la revendication 1 ou 2, dans laquelle l'agent diluant est choisi dans le groupe comprenant le lactose, la cellulose microcristalline, le phosphate de calcium dibasique et le mannitol.

4. La formulation pharmaceutique pour son utilisation selon une quelconque des revendications 1 à 3, dans laquelle l'agent absorbant est choisi dans le groupe comprenant le phosphate de calcium dibasique, le silicate d'aluminium et de magnésium, le dioxyde de silicium colloïdal et la cellulose microcristalline.

5. La formulation pharmaceutique pour son utilisation selon une quelconque des revendications 1 à 4, dans laquelle l'agent de désintégration est choisi dans le groupe comprenant le croscarmellose de sodium, l'amidon de maïs et la crospovidone.

6. La formulation pharmaceutique pour son utilisation selon une quelconque des revendications 1 à 5, dans laquelle l'agent lubrifiant est choisi dans le groupe comprenant le stéarate de magnésium, le talc et l'acide stéarique.

7. La formulation pharmaceutique pour son utilisation selon une quelconque des revendications 1 à 6, dans laquelle l'agent de glissement est du dioxyde de silicium colloïdal.

8. La formulation pharmaceutique pour son utilisation selon une quelconque des revendications 1 à 7, dans laquelle l'enzyme digestive est l'α-D-galactosidase présentant une énergie enzymatique de 450 Ugal.

9. Un procédé de préparation d'une composition pharmaceutique selon une quelconque des revendications 1 à 8, comprenant :
le mélange du modificateur de motilité intestinale, de l'agent empêchant la rétention de gaz et de l'enzyme digestive, avec une solution de liaison pour humidifier le modificateur de motilité intestinale et l'enzyme digestive; le broyage, le séchage et le tamisage du mélange ; pour obtenir une formulation pharmaceutique adaptée pour une administration orale.

10. Le procédé selon la revendication 9, dans lequel les composés sont séchés à une température comprise entre 30 et 60°C et la composition atteint une humidité résiduelle finale non supérieure à 5%.

11. Le procédé selon la revendication 10, dans lequel le mélange est tamisé à travers un maillage de 420 à 2 000 microns.
